# EUROPÄISCHE PATENTSCHRIFT

(11) **EP 1 761 218 B1**
(45) Veröffentlichungstag und Bekanntmachung des Hinweises auf die Patenterteilung: **29.06.2011**
(21) Anmeldenummer: 05753237.6
(22) Anmeldetag: 17.05.2005
(51) Int. Cl.: A61F 2/64

(54) **PROTHESENGELENK**
PROSTHETIC JOINT
PROTHESE D'ARTICULATION

(30) Priorität: 19.05.2004 DE 202004008157 U
(43) Veröffentlichungstag der Anmeldung: 14.03.2007
(73) Patentinhaber: medi GmbH & Co. KG, 95448 Bayreuth (DE)
(72) Erfinder: SHEN, Hsin, Fa, Taipei (TW)
(74) Vertreter: Blaumeier, Jörg
(86) Internationale Anmeldenummer: PCT/DE2005/000894
(87) Internationale Veröffentlichungsnummer: WO 2005/112837

(56) Entgegenhaltungen:
- EP-A- 0 095 872
- EP-A- 1 166 726
- DE-C- 342 833
- DE-C- 362 891
- DE-C- 726 228
- DE-U1- 20 119 049
- US-A- 3 408 660

## Beschreibung

Die vorliegende Erfindung betrifft eine Prothesengelenk mit den Merkmalen des Oberbegriffs des Anspruchs 1.

Ein Prothesengelenk für den Einsatz als künstliches Knie, bestehend aus einem Knieoberteil, einem Knieunterteil und einer diese verbindenden Klemmfaust, wobei das Knieoberteil um eine Drehachse der Klemmfaust drehbar angeordnet ist und die Drehung gegen die Kraft eines im Knieunterteil angeordneten elastischen Elements erfolgt ist zum Beispiel aus der EP 1 166 726 A1 oder der DE 201 19 049 U1 bekannt. Die EP 1 166 726 A1 zeigt ferner sämtliche Merkmale des Oberbegriffs des Anspruchs 1. Durch diese Mechanik erfolgt eine abgebremste Flexion, während die Extension vergleichsweise ungebremst ablaufen kann.

Aufgabe der vorliegenden Erfindung ist es, ein derartiges Prothesengelenk dahingehend fortzubilden, dass es ohne großen Aufwand in bestimmten Stellungen, z.B. in der Flexionsstellung fixiert werden kann. Dies ist für frisch Amputierte, die noch nicht in der Lage sind, ein Kniegelenk sicher zu steuern, von großem Vorteil. Bisher war die Praxis so, dass nacheinander zwei verschiedene Kniegelenke verwendet wurden, d.h. die Prothesen mussten umgebaut werden. Sowohl die Verwendung zweier Kniegelenke, wie auch der Umbauvorgang waren kostspielig, so dass es wünschenswert war, beide Funktionen in einem Kniegelenk zu kombinieren.

Diese Aufgabe wird mit den Merkmalen des kennzeichnenden Teils des Anspruchs 1 gelöst. Fortbildungen und vorteilhafte Ausführungen der Erfindung sind im Folgenden umfasst.

Erfindungsgemäß ist ein Prothesengelenk für den Einsatz als künstliches Knie, bestehend aus einem Knieoberteil, einem Knieunterteil und einer diese verbindenden Klemmfaust, wobei das Knieoberteil um eine Drehachse der Klemmfaust drehbar angeordnet ist und die Drehung gegen die Kraft eines im Knieunterteil angeordneten elastischen Elements erfolgt, wobei zwischen dem Knieoberteil und der Klemmfaust lösbare Feststellmittel für die Fixierung wenigstens einer Drehposition vorhanden sind, dadurch gekennzeichnet, dass die Feststellmittel eine an dem Knieoberteil angelenkte Klinke sind, die in eine Ausnehmung der Klemmfaust einrückbar ist. Bei der gezeigten Ausführung weist die Klinke Mittel zur Fixierung ihrer Position an dem Knieoberteil auf. Vorteilhafterweise ist die Klinke durch ein elastisches Element, z.B. eine Feder, in Verriegelungsstellung beaufschlagt. Vorzugsweise weist die Klinke einen Betätigungshebel auf. Die fixierbare Drehposition ist beispielsweise die Extensionsstellung.

Nach der bevorzugten Ausführung der Erfindung ist die Drehachse der Klemmfaust exzentrisch und drehbar mit wenigstens einer Vorbringerschubstange verbunden, die auf einem Federkolben einer in dem Knieunterteil gelagerten Feder angelenkt ist. Die Feder ist vorzugsweise eine mechanische Druckfeder, deren Federweg und Kennlinie durch eine Schraubvorrichtung einstellbare Feder.

Zwischen der Klemmfaust und dem Knieunterteil ist vorzughsweise ein verstellbarer Einstellkeil vorhanden.

Im Folgenden wird die Erfindung anhand von Zeichnung en beispielhaft näher beschrieben.
Fig. 1 eine Seitenansicht eines Prothesengelenks in Extensionsstellung;
Fig. 2 eine Rückansicht des Prothesengelenks von Fig. 1;
Fig. 3 einen teilweisen Schnitt in der Hochebene durch das Prothesengelenk von Fig. 1;
Fig. 4 das Prothesengelenk von Fig. 1 in der Flexionsstellung.

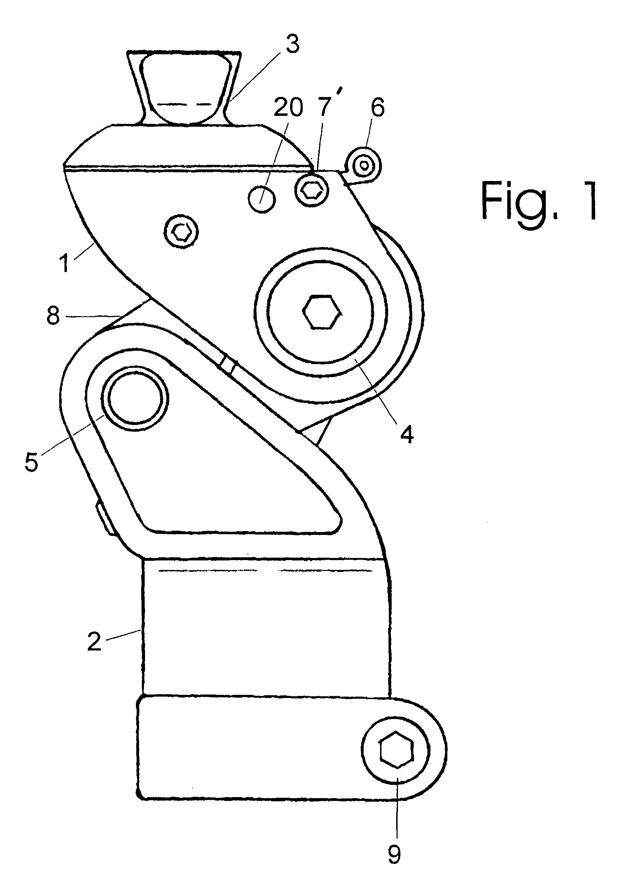

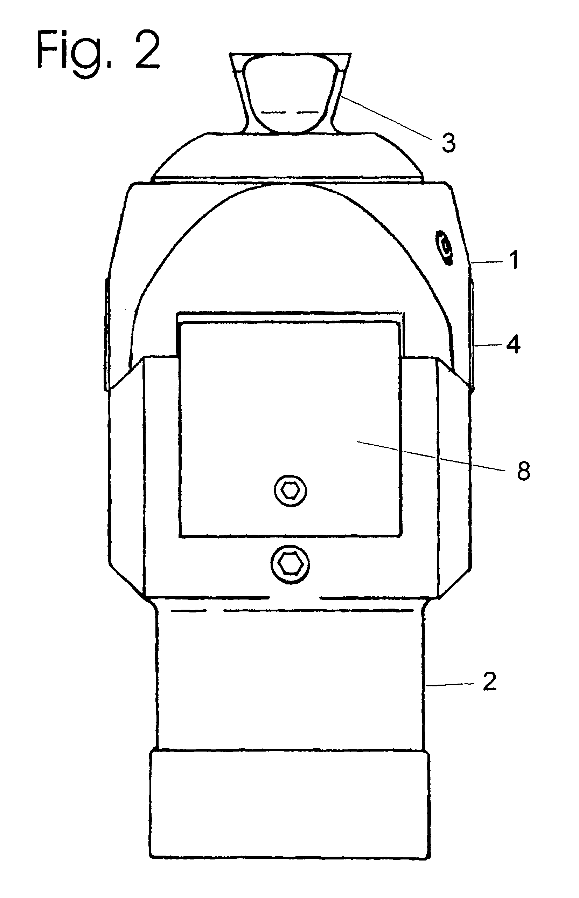

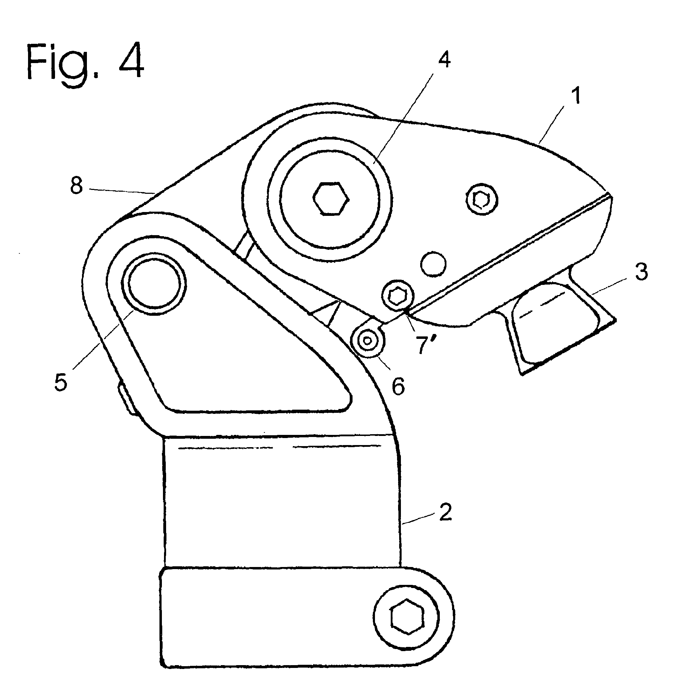

Fig. 1, 2 und 3 zeigen das Prothesengelenk von außen. Es besteht aus einem Knieoberteil 1, einem Knieunterteil 2 und einem diese beiden verbindenden Klemmfaust 8. Das Knieoberteil 1 besitzt an seinem oberen Ende in bekannter Weise eine Pyramide 3 für den Anschluss an einen Prothesenschaft (nicht gezeigt). Das Knieunterteil 2 besitzt am unteren Ende eine Schelle mit einer Klemmschraube9 für den Anschluss eines Prothesenrohres (nicht gezeigt). Die Klemmfaust 8 ist zwischen zwei Schenkeln des Knieierunterteils 2 auf einer Bremsanlenkachse 5 gelagert. Das Knieoberteil 1 ist auf einer Drehachse 4 der Klemmfaust 8 gelagert und ist zwischen der Extensionsstellung (Fig. 1) und der Flexionsstellung (Fig. 4) verschwenkbar.

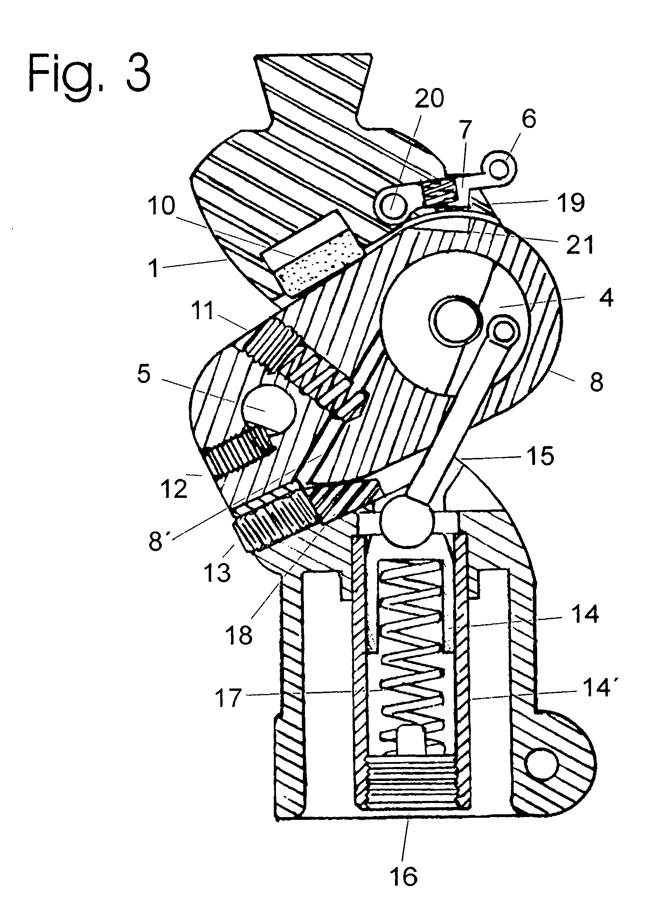

Die Funktionsweise des Prothesengelenks wird nun anhand von Fig. 3 erläutert. Die Klemmfaust 8 ist mit einem Schlitz 8' ausgeführt und auf der Drehachse 4 drehbar gelagert, die in drehfest seitlichen Schenkeln des Knieoberteils 1 gehalten ist. In der Extensionsstellung drückt das Gewicht des Benutzers auf das Knieoberteil 1 und einen in einer Ausnehmung, die zu der Oberseite der Klemmfaust 8 hin offen ist, gehaltenen Extensionsanschlag 10. Die ausgeübte Kraft wirkt schließend auf den Schlitz 8', so dass sich die Klemmfaust 8 fester um die Drehachse 4 schließt und mit dieser klemmend in Eingriff kommt. Zur Einstellung der Klemmwirkung, den Schwellenwert der Auflast und die Progression der Bremswirkung sind in an sich bekannter Weise eine Bremsregulierschraube 11 mit Feder, eine Fixierungsschraube 12 für die Anlenkachse 5 und ein durch eine weitere Schraube 13 einstellbarer Keil 18 vorhanden. Zwischen der Drehachse 4 und einer in dem Knieunterteil angeordneten Dämpfungsanordnung aus einem Kolben 14', einem Zylinder 14, einer diesen beaufschlagenden Spiralfeder 17 und einer Einstellschraube 16 ist eine Vorbringerschubstange 15 angeordnet. Die Vorbringerschubstange 15 ist an ihrem unteren Ende mit dem Zylinder 14 verbunden und mit ihrem oberen Ende exzentrisch an der Drehachse 4 angelenkt. Wird das Prothesengelenk von der Extensionsstellung (Fig. 1, 2 und 3) in die Flexionsstellung (Fig. 4) gebracht (beim Anwinkeln des Unterschenkels), so wird die Klemmwirkung der Klemmfaust 8 aufgehoben und das Knieoberteil 1 dreht sich im Uhrzeigersinn um die Drehachse 4. Dies erfolgt gegen die Kraft der Feder 17 der Dämpfungsanordnung, die von der Vorbringerschubstange zusammengedrückt wird. Wird das Prothesengelenk wieder in die Flexionsstellung gebracht, so wirkt die Dämpfungsanordnung als Vorbringer und die Vorbringerschubstange 15 drückt die Drehachse 4 mit dem Knieoberteil 1 wieder zurück in die Flexionsstellung.

Soll nun das Prothesengelenk für eine "erste" Prothese nach der Amputation verwendet werden, so kann das Knieoberteil 1 gegenüber der Klemmfaust 8 durch eine Sperrvorrichtung fixiert werden. Die Sperrvorrichtung besteht aus einer Klinke 7, die schwenkbar um einen Bolzen 20 über der Klemmfaust 8 an dem Knieoberteil 1 gelagert ist. Die Klinke 7 kann in der Extensionsstellung in eine Ausnehmung 21 der Klemmfaust 8 eingerückt werden und sperrt so die Bewegung des Knieoberteils 1. Die Klinke 7 besitzt einen nach außen geführten Betätigungshebel 6 und ist von einer Feder 19 in Sperrstellung beaufschlagt. Zur Fixierung der Klinke in der eingerückten oder gelösten Position ist eine Arretierungsschraube 7' (Fig. 1) vorhanden.

## Patentansprüche

1. Prothesengelenk für den Einsatz als künstliches Knie, bestehend aus einem Knieoberteil (1), einem Knieunterteil (2) und einer diese verbindenden Klemmfaust (8), wobei das Knieoberteil (1) um eine Drehachse (4) der Klemmfaust (8) drehbar angeordnet ist und die Drehung gegen die Kraft eines im Knieunterteil (3) angeordneten elastischen Elements erfolgt,
wobei zwischen dem Knieoberteil (1) und der Klemmfaust (8) lösbare Feststellmittel für die Fixierung wenigstens einer Drehposition vorhanden sind,
**dadurch gekennzeichnet,**
**dass** die Feststellmittel eine an dem Knieoberteil (1) angelenkte Klinke (7) sind, die in eine Ausnehmung (21) der Klemmfaust (8) einrückbar ist.

2. Prothesengelenk nach Anspruch 1,
**dadurch gekennzeichnet,**
**dass** die Klinke (7) Mittel zur Fixierung ihrer Position an dem Knieoberteil (1) aufweist.

3. Prothesengelenk nach Anspruch 1 oder 2,
**dadurch gekennzeichnet,**
**dass** die Klinke (7) durch ein elastisches Element in Verriegelungsstellung beaufschlagt ist.

4. Prothesengelenk nach Anspruch 1, 2 oder 3,
**dadurch gekennzeichnet,**
**dass** die Klinke (7) einen Betätigungshebel (6) aufweist.

5. Prothesengelenk nach einem der vorhergehenden Ansprüche,
**dadurch gekennzeichnet,**
**dass** die fixierbare Drehposition die Extensionsstellung ist.

6. Prothesengelenk nach einem der vorhergehenden Ansprüche,
**dadurch gekennzeichnet,**
**dass** die Drehachse (4) der Klemmfaust (8) exzentrisch und drehbar mit wenigstens einer Vorbringerschubstange (15) verbunden ist, die auf einem Federkolben einer in dem Knieunterteil (3) gelagerten Feder (17) angelenkt ist.

7. Prothesengelenk nach Anspruch 6,
**dadurch gekennzeichnet,**
**dass** die Feder (17) eine einstellbare Feder ist.

8. Prothesengelenk nach einem der vorhergehenden Ansprüche,
**dadurch gekennzeichnet,**
**dass** zwischen der Klemmfaust (8) und dem Knieunterteil (2) ein verstellbarer Einstellkeil (18) vorhanden ist.

## Claims

1. A prosthetic joint for use as an artificial knee, comprising an upper-knee part (1), a lower-knee part (2) and a clamping socket (8) connecting the latter, wherein the upper-knee part (1) is arranged in a manner rotatable about an axis of rotation (4) of the clamping socket (8), and the rotation takes place against the force of an elastic element disposed in the lower-knee part (2),
wherein fixing means for fixing at least one rotational position are provided between the upper-knee part (1) and the clamping socket (8),
**characterised in that**
the fixing means is a ratchet (7) articulated on the upper-knee part (1), which can engage in a recess (21) of the clamping socket (8).

2. The prosthetic joint according to claim 1,
**characterised in that**
the ratchet (7) provides means for fixing its position on the upper-knee part (1).

3. The prosthetic joint according to claim 1 or 2,
**characterised in that**
the ratchet (7) is biased into the locking position by an elastic element.

4. The prosthetic joint according to claim 1, 2 or 3,
**characterised in that**
the ratchet (7) provides an activation lever (6).

5. The prosthetic joint according to any one of the preceding claims,
**characterised in that**
the fixable rotational position is the extended position.

6. The prosthetic joint according to any one of the preceding claims,
**characterised in that**
the axis of rotation (4) of the clamping socket (8) is eccentric and is connected in a rotatable manner to at least one forward-displacement connecting rod (15), which is articulated on a spring-loaded piston of a spring (17) mounted in the lower-knee part (2).

7. The prosthetic joint according to claim 6,
**characterised in that**
the spring (17) is an adjustable spring.

8. The prosthetic joint according to any one of the preceding claims,
**characterised in that**
an adjustable adjustment wedge (18) is provided between the clamping socket (8) and the lower knee part (2).

## Revendications

1. Articulation prothétique, destinée à être utilisée sous forme de prothèse du genou, formée par une partie de genou supérieure (1) et une partie de genou inférieure (2) et par une chape de serrage (8) reliant ces dernières, la partie de genou supérieure (1) étant montée rotative autour d'un axe de rotation (4) de la chape de serrage (8) et la rotation s'effectuant à l'encontre de la force d'un élément élastique disposé dans la partie de genou inférieure (2),
des moyens de blocage amovibles étant présents entre la partie de genou supérieure (1) et la chape de serrage (8) pour la fixation dans au moins une position de rotation,
**caractérisée en ce que** les moyens de blocage sont un cliquet (7), qui est articulé contre la partie de genou supérieure (1) et qui peut s'enclencher dans un évidement (21) de la chape de serrage (8).

2. Articulation prothétique selon la revendication 1, **caractérisée en ce que** le cliquet (7) comporte des moyens pour le fixer dans une position sur la partie de genou supérieure (1).

3. Articulation prothétique selon la revendication 1 ou 2, **caractérisée en ce que** le cliquet (7) est sollicité dans la position de verrouillage par un élément élastique.

4. Articulation prothétique selon la revendication 1, 2 ou 3, **caractérisée en ce que** le cliquet (7) comporte un levier d'actionnement (6).

5. Articulation prothétique selon l'une quelconque des revendications précédentes, **caractérisée en ce que** la position de rotation pouvant être immobilisée est la position en extension.

6. Articulation prothétique selon l'une quelconque des revendications précédentes, **caractérisée en ce que** l'axe de rotation (4) de la chape de serrage (8) est relié, de manière excentrée et rotative, à au moins une bielle de poussée (15), qui est articulée sur un piston sollicité par un ressort (17) logé dans la partie de genou inférieure (2).

7. Articulation prothétique selon la revendication 6, **caractérisée en ce que** le ressort (17) est un ressort réglable.

8. Articulation prothétique selon l'une quelconque des revendications précédentes, **caractérisée en ce qu'**une cale de réglage (18) réglable est présente entre la chape de serrage (8) et la partie de genou inférieure (2).
